# NEW EUROPEAN PATENT SPECIFICATION

(11) **EP 1 143 924 B2**
(45) Date of publication and mention of the opposition decision: **26.11.2008**
(45) Mention of the grant of the patent: 26.10.2005
(21) Application number: 99901441.8
(22) Date of filing: 12.01.1999
(51) Int. Cl.: A61Q 19/10, A61K 8/35

(54) **EXOTHERMIC EFFERVESCENT COMPOSITION FOR IMPROVED FRAGRANCE DISPERSION**
EXOTHERMISCHE BRAUSEZUBEREITUNG FÜR DIE VERBESSERTE DISPERSION DES AROMAS
COMPOSITION EFFERVESCENTE EXOTHERME AMELIORANT LA DIFFUSION D'UN PARFUM

(43) Date of publication of application: 17.10.2001
(73) Proprietor: Phyzz, Inc., Cincinnati, OH 45242 (US)
(72) Inventor: NEEDLEMAN, Norman, Ivoryton, CT 06442 (US); RAU, Allen, Cincinnati, OH 45249 (US)
(74) Representative: Dunlop, Hugh Christopher
(86) International application number: PCT/US1999/000641
(87) International publication number: WO 2000/041673

(56) References cited:
- EP-A- 0 529 931
- EP-A- 0 687 464
- DE-A- 19 757 059
- JP-A- 61 078 717
- US-A- 4 592 855
- US-A- 5 602 178
- US-A- 5 824 629
- CHEMICAL ABSTRACTS, vol. 3, no. 126, 20 January 1997 (1997-01-20) Columbus, Ohio, US; abstract no. 36884, YOSHIOKA, TOSHIO AND HIRAIWA, HIROMI: "Effervescent cosmetic packs containing exothermic substances" XP002081132 & JP 08 268828 A (SHISEIDO CO LTD JAPAN) 15 October 1996 (1996-10-15)

## Description

### TECHNICAL FIELD

This invention relates to tablets containing a volatile material such as a fragrance agent and more particularly, to tablets which promote and enhance the release of the volatile fragrance material when added to water utilizing a combination of effervescent and exothermic reactions.

### BACKGROUND

Fragrances are used in many products to improve their acceptance by consumers. For example, most personal products such as moisturizers, cleansers, and even household products such as laundry detergents use fragrances to improve their consumer acceptability. There have also been many studies conducted which have speculated that aroma greatly influences human psychology and physiology, and the term "aromatherapy" has been used to describe the beneficial properties which can be achieved using fragrances. In U.S. Patent No. 5,238,915, various aromatic compositions or perfumes are discussed in relation to aromatherapy. In the '915, the prolonged release of perfume is accomplished by a pH adjusting tablet to provide a variable release of the perfume.

Volatile fragrance materials which are inhaled are included in many medications for treating conditions such as congested sinuses and coughing. For example, menthol and camphor are recognized by the United States FDA as safe and effective for the treatment of these conditions.

Effervescent compositions are known. These compositions generally combine carbonate salts such as sodium carbonate and/or sodium bicarbonate with acidic materials such as citric, tartaric, or fumaric acid in a way that carbon dioxide is released when the product is placed in water. These products must be packaged in ways that prevent unintended contact with water so that premature reaction is avoided. Even contact with humidity in the air must be prevented during manufacture and storage as this could detrimentally effect the effervescent properties. These effervescent compositions have been used with compounds such as aspirin or acetaminophen to treat headaches and stomach upset, been added with bleach and surfactants to clean dentures such as in U.S. Patent No. 5,384,062, and included with fragrance and moisturizers to provide bath salts.

Various compositions are known which have exothermic heats of solution. A number of these are listed in Lange's Handbook of Chemistry, 11th edition, in Table 9-6 (P9-107). The greater the value of the heat of solution, the more heat is liberated per gram-mole of the substance. Thus, materials with higher heats of solution are able to raise the temperature of a given amount of water higher than compounds with lower heats of solution. In U.S. Patent No. 4,818,518, a dentifrice composition is described which provides a warming sensation using an exothermic reaction.

However, there still exists in the art a need for compositions which enhance the ability to promote release of volatile materials when placed in water.

### SUMMARY OF THE INVENTION

It is an object of the present invention to provide an aroma releasing tablet as in claim 1 herein below.

It is a further object of the present invention to provide a method of releasing an aroma as in claim 7 herein below.

These and other objects of the present invention are achieved by an aromatic tablet comprising an effervescent agent, an exothermic agent and a fragrance agent, compounded into an essentially anhydrous form, the effervescent agent and exothermic agent promoting release of the volatile agent once the composition is placed in water.

Utilizing this combination of materials promotes and enhances release of the fragrance agent beyond that achievable by using either the effervescent or exothermic agent alone.

### DETAILED DESCRIPTION OF THE INVENTION

The effervescent agent is preferably provided by combining an alkaline carbonate salt or a combination of salts such as sodium carbonate with an acid such as citric acid, malic acid, fumaric acid, succinic acid or tartaric acid. More than one acid can be used, if desired. The exact combination of acidic and alkaline materials can be varied in order to give an acidic or alkaline pH. They can also be varied to enhance the stability and physical properties of the finished product.

Among the usable alkaline carbonate salts are salts such as sodium bicarbonate, sodium carbonate, sodium sesquicarbonate, potassium bicarbonate, potassium carbonate, potassium sesquicarbonate, magnesium carbonate, ammonium bicarbonate, ammonium carbonate, ammonium sesquicarbonate, and calcium carbonate. These can be used alone or in combination with each other. Of course, other effervescent agents may be used in the present invention.

Acids usable in the invention include formic acid, acetic acid, propanoic acid, butyric acid, valeric acid, oxalic acid, malonic acid, tartaric acid, succinic acid, glutaric acid, adipic acid, glycolic acid, aspartic acid, pimelic acid, fumaric acid, maleic acid, phthalic acid, isophthalic acid, terphthalic acid, glutamic acid, lactic acid, hydroxy acrylic acid, alpha hydroxy butyric acid, glyceric acid, tartronic acid, hydroxy benzoic acid, citric acid, salicylcic acid, gallic acid, mandelic acid, tropic acid, ascorbic acid, gluconic acid, cinnamic acid, benzoic acid, phenylacetic acid, nicotinic acid, kainic acid, sorbic acid, pyrrolidone carboxylic acid, trimellitic acid, benzene sulfonic acid, toluene sulfonic acid, potassium dihydrogen phosphate, sodium sulfite, sodium dihydrogen phosphate, potassium sulfite, sodium pyrosulfite, acidic sodium hexametaphosphate, acidic potassium hexametaphosphate, acidic sodium pyrophosphate, acidic potassium pyrophosphate, sulfamic acid and phosphoric acid. These can be used alone or in combination with each other. Of course, other acids may be used in the present invention.

The exothermic agent is chosen from magnesium chloride, magnesium sulfate, ferric chloride and aluminum chloride. The material has an exothermic heat of solution greater than about 10 kcal/gram-mole.

The volatile fragrance materials used in the tablet are ones which are able to emanate aroma or fragrance. Examples of such perfumes include natural perfumes which originate from natural plants and animals and whose aromatic ingredients are collected therefrom by physical and chemical treatments such as steam distillation, extraction and the like, chemical substances derived from resources such as coal, petroleum, natural gas, oils and fats, and perfumes prepared through chemical reactions of the natural perfumes and isolated perfumes, such as of oxidation, reduction, condensation, hydrolysis, substitution, addition and transition.

Specific examples of the perfumes include animal perfumes such as musk oil, civet, castreum, ambergris, plant perfumes such as sandalwood oil, neroli oil, bergamot oil, lemon oil, lavender oil, sage oil, rosemary oil, peppermint oil, eucalyptus oil, menthol, camphor, verbena oil, citronella oil, cauout oil, salvia oil, clove oil, chamomille oil, sandalwood oil, costus oil, labdanum oil, broom extract, carrot seed extract, jasmine extract, minmosa extract, narcissus extract, olibanum extract, rose extract, and chemical substances such as acetophenonene, dimethylinadane derivatives, naphthaline derivatives, allyl caprate, α-amylcinnamic aldehyde, anethole, anisaldehyde, benzyl acetate, benzyl alcohol, benzyl propionate, bomeol, cinnamyl acetate, cinnamyl alcohol, citral citronnellal, cumin aldehyde, cyclamen aldehyde, decanol, ethyl butyrate, ethyl caprate, ethyl cinnamate, ethyl vanillin, eugenol, geraniol, hexenol, α-hexylcinnamic aldehyde, hydroxycitrolnellal, indole, iso-amyl acetate, iso-amyl acetate, iso-amyl iso-valeratek iso-eugenol, linalol, linalyl acetate, p-methylacetophenone, methyl anthranilate, methyl dihydroasmonate, methyl eugenol, methyl-β-naphthol ketone, methylphenylcarbinyl acetate, musk ketol, musk xylol, 2,56-nanodinol, γ-nanolactone, phenylacetoaldehydodimethyl acetate, β-phenylethyl alcohol, 3,3,5-trimethylcyclohexanol, γ-undecalactone, undecenal, vanillin and mixtures thereof These perfumes may be used singly or in combination of two or more, and of course, other perfumes may be used in the present invention.

The tablet of the invention may further comprise arbitrary ingredients other than the above-stated essential ingredients, in amounts not impeding the effect of the aromatic tablet. Such arbitrary ingredients include, for example, saccharides, surface active agents, binders, buffers, oils and fats, high molecular weight compounds and the like.

Several exemplary products were prepared by first providing an oil premix composition of 6.3% wt. eucalyptus oil, 12.7% wt. menthol, 25.3% wt. camphor, adsorbed on to 55.7% wt. maltodextrin, mixing with sodium bicarbonate in a glass mortar. Other ingredients may be used, for example, in formula 4, the oil premix was combined with a portion of sorbitol in a glass mortar. Sorbitol is used as a binder. Of course, numerous other volatile materials, including but not limited to fragrance agents and inhalants, may be used in the invention. PEG-150, a polyethylene glycol material, is used as another binder, but, various other formulating agents may be used in the inventive composition. Each mixture was then blended with all of the remaining ingredients, except for the PEG-150, in a V-blender. After 5 minutes of mixing, the PEG-150 was added to the V-blender. Mixing was continued an additional three minutes. The resulting powder mixture was discharged into a plastic bag and sealed until it was compressed into 30 gram tablets using a Colton press with 1.8366" rounded square corner, concave face, beveled edge punches. Of course, various tablet sizes can be used with the present invention, ranging from approx. 2 to approx. 100 grams per tablet.

The following charts illustrates the superiority of the invention.

| PERCENTAGE FORMULAS (by weight) | | | | | |
|---|---|---|---|---|---|
| | Formula 1 | Formula 2 | Formula 3 | Formula 4 | Formula 5 |
| Citric Acid | 25.55 | 25.55 | | 25.55 | 25.55 |
| Na₂CO₃ | 12.70 | 12.70 | 12.70 | | 12.70 |
| Na₂HCO₃ | 13.00 | 13.00 | 13.00 | | 13.00 |
| Oil Premix | 3.40 | 3.40 | 3.40 | 3.40 | 3.40 |
| MgCl₂ | 28.85 | | 28.85 | 28.85 | |
| MgSO₄ | | | | | 28.85 |
| PEG-150 | 0.50 | 0.50 | 0.50 | 0.50 | 0.50 |
| Sorbitol | 16.00 | 44.85 | 41.55 | 41.70 | 16.00 |
| TOTAL | 100.00 | 100.00 | 100.00 | 100.00 | 100.00 |

Formula 1 includes both an effervescent agent and an exothermic agent. Formula 2 has only an effervescent agent, Formulas 3 and 4 have an exothermic agent but no effervescent agent. Formula 5 has both.

These tablets were evaluated on the following parameters: thickness, hardness, dissolution time, temperature increase, and fragrance impact. Thickness and hardness are standard measures of tablet properties. They were evaluated using calipers and a Key hardness tester. It is generally desired to have hardness in excess of 6 kp. The dissolution time and temperature rise were measured by placing a tablet in 50 ml of 25°C water. Fragrance impact is a subjective evaluation. Data are shown below:

| | Formula 1 | Formula 2 | Formula 3 | Formula 4 | Formula 5 |
|---|---|---|---|---|---|
| Thickness (mm) | 11.3 | 12.8 | 12.8 | 12.7 | 11.0 |
| Hardness (kp) | 12.7 | 16.1 | 11.9 | 16.7 | 9.8 |
| Dissolution time (min:sec) | 6:57 | 10:03 | 29:45 | 40:32 | 9:35 |
| Temp Rise | 29°C | -5°C | 19°C | 9°C | 10°C |
| Fragrance Lift | Very good | Poor | Poor | Poor | Good |

Formulae 1 and 5 which include the inventive combination show that combining an effervescent agent with an exothermic agent capable of generating heat upon exposure to water gives the best dispersion and release of the volatile components. Formula 2 shows that when the heat generating material is omitted, the effervescent action actually cools the resulting solution during the reaction. Formulae 3 and 4 show that when either member of the effervescent acid/carbonate salt couple is left out, the warming effect is not sufficient to adequately disperse the volatile materials on its own.

These data clearly show that the combined effects of the effervescent reaction and exothermic reaction combine in an unexpected way to dramatically increase the lift and release of the volatile materials. Preferably, a ratio of effervescent (acid plus carbonate) to exothermic agent would be about 1 to 10, to 10 to 1, as confirmed by the following examples.

Formulations were compounded using the same method described earlier. The oil premix is the same as in previous examples: 6.3% eucalyptus oil, 12.7% menthol, 25.3% camphor adsorbed on to 55.7% maltodextrin. 30 gram tablets were prepared using a 2.25 inch diameter round die and a manually operated Carver Press. 12000 psi pressure was applied to the punches for 3 minutes to form the tablets. Since this method of forming the tablets is slightly different than that used in the previous experiments (therefore resulting in different size and thickness), tablets of formula 1 were produced again as a point of reference. The fact that the temperature rise data on these Formula 1 tablets is substantially the same as the data from the previous experiments confirms that this physical change is not significant.

| PERCENTAGE FORMULAS (by weight) | | | | | |
|---|---|---|---|---|---|
| | Formula 1 | Formula 6 | Formula 7 | Formula 8 | Formula 9 |
| Citric Acid | 25.55 | 15.33 | 20.44 | 36.28 | 3.63 |
| Na₂CO₃ | 12.70 | 7.62 | 10.16 | 18.03 | 1.85 |
| Na₂HCO₃ | 13.00 | 7.80 | 10.40 | 18.46 | 3.40 |
| Oil Premix | 3.40 | 3.40 | 3.40 | 3.40 | 3.40 |
| MgCl₂ | 28.85 | 49.35 | 39.10 | 7.32 | 72.82 |
| PEG-150 | 0.50 | 0.50 | 0.50 | 0.50 | 0.50 |
| Sorbitol | 16.00 | 16.00 | 16.00 | 16.00 | 16.00 |
| TOTAL | 100.00 | 100.00 | 100.00 | 100.00 | 100.00 |

| | Formula 1 | Formula 6 | Formula 7 | Formula 8 | Formula 9 |
|---|---|---|---|---|---|
| Effervescent/ Exothermic ratio | 5/3 | 3/5 | 1/1 | 10/1 | 1/10 |
| Thickness (mm) | 8.3 | 7.8 | 8.4 | 8.7 | 7.3 |
| Hardness (kp) | 17.2 | 16.7 | 20.2 | 6.0 | 19.6 |
| Dissolution time (min:sec) | 4:10 | 6:20 | 6:38 | 3:05 | 15.00 |
| Temp Rise | 27°C | 53°C | 42°C | 0°C | 44°C |
| Fragrance lift | Very good | Excellent | Excellent | Very good | Very good |

This data confirms that the effervescent to exothermic ratios of 10/1 to 1/10 give the inventive products acceptable performance. In fact, it can be seen that manipulation of this ratio can be used to tailor the dissolution time of the product. Thus, if one wanted to extend the release of volatile material, the effervescent to exothermic ratio could be decreased without causing significant deterioration of performance.

While preferred embodiments of the inventions have been shown and described, these are merely exemplary and one skilled in the art may vary these parameters without varying from the scope of the inventions.

## Claims

1. An aroma releasing tablet comprising:
an effervescent agent; said effervescent agent being a composition that effervesces upon contact with water;
an exothermic agent; said exothermic agent being a composition that exhibits an exothermic reaction upon contact with water and having a heat of solution greater than about 10 kcal/gram.mole and being selected from magnesium chloride, magnesium sulfate, ferric chloride, and aluminium chloride; and
a volatile fragrance agent, the effervescent agent and exothermic agent provided in a ratio sufficient to promote release of the volatile agent, when the composition is placed in water, the agents being in an essentially anhydrous form, wherein the ratio of effervescent agent to exothermic agent is from about 1: 10 to about 10:1.

2. The tablet of claim 1 wherein the effervescent agent is a combination of an alkaline carbonate salt and an acid.

3. The tablet of claim 2 wherein the alkaline carbonate salt is selected from the group consisting of sodium carbonate, sodium bicarbonate, sodium sesquicarbonate, potassium bicarbonate potassium carbonate, potassium sesquicarbonate, magnesium carbonate, ammonium bicarbonate, ammonium carbonate, ammonium sesquicarbonate, calcium carbonate and combinations thereof.

4. The tablet of claim 2 wherein the acid is selected from the group consisting citric acid, malic acid, fumaric acid, succinic acid, tartaric acid, formic acid, acetic acid, propanoic acid, butyric acid, valeric acid, oxalic acid, malonic acid, tartaric acid, succinic acid, glutaric acid, adipic acid, pimelic acid, fumaric acid, maleic acid, phthalic acid, isophthalic acid, terphthalic acid, glutamic acid, lactic acid, hydroxy acrylic acid, alpha hydroxy butyric acid, glyceric acid, tartronic acid, hydroxy benzoic acid, citric acid,salicylic acid, gallic acid, mandelic acid, tropic acid, ascorbic acid, gluconic acid, cinnamic acid, benzoic acid,phenylacetic acid, nicotinic acid, kainic acid, sorbic acid, pyrrolidonecarboyxlic acid,trimellitic acid, benzene sulfonic acid, totuene sulfonic acid, potassium dihydrogen phosphate, sodium sulfite, sodium dihydrogen phosphate, potassium sulfite, sodium pyrosulfite, acidic sodium hexametaphosphate, acidic potassium hexametaphosphate, acidic sodium pyrophosphate, acidic potassium pyrophosphate,sulfamic aicd, phosphoric acid and combinations thereof.

5. The tablet of claim 1 wherein the volatile agent is selected from the group consisting of musk oil, civet, castreum, ambergris, plant perfumes such as sandalwood oil, neroli oil, bergamot oil, lemon oil, lavender oil, sage oil, rosemary oil, peppermint oil, eucalyptus oil, menthol, camphor, verbena oil, citronella oil, cauout oil, salvia oil, clove oil, camomille oil, sandalwood oil, costus oil, labdanum oil, broom extract, carrot seed extract, jasmine extract, minmosa extract, narcissus extract, olibanum extract, rose extract and the like, and chemical substances such as acetophenonene,dimethylinadane derivatives, naphthaline derivatives, allyl caprate, a-amylcinnamic aldehyde, anethole, anisaldehyde, benzyl acetate, benzyl alcohol, benzylpropionate, borneol, cinnamyl acetate, cinnamyl alcohol, citralcitronnellal, cumin aldehyde, cyclamen aldehyde, decanol, ethyl butyrate, ethyl caprate, ethyl cinnamate, ethyl vanillin, eugenol, geraniol, hexenol, a-hexylcinnamic aldehyde, hydroxycitrolnellal, indole, iso-amyl acetate, iso-amyl iso-valeratek iso-eugenol, linalol, linalyl acetate, p-methylacetophenone, methylanthranilate, methyl dihydroasmonate, methyl eugenol,methyl-ss-naphthol ketone, methylphenhlcarbinyl acetate, musk ketol, musk xylol, 2,5,6-nanodinol, y-nanolactone, phenylacetoaldehydodimethyl acetate,P-phenylethyl alcohol, 3,3,5-trimethylcyclohexanol, yundecalactone, undecenal, vanillin and mixtures thereof.

6. The tablet of claim 1 wherein the volatile agent is selected from the group consisting of eucalyptus oil, menthol, camphor and combinations thereof.

7. A method of releasing an aroma comprising: providing an aroma releasing tablet having a effervescent agent being a composition that effervesces upon contact with water, an exothermic agent being a composition that exhibits an exothermic reaction upon contact with water, said exothermic agent being selected from magnesium chloride, magnesium sulfate, ferric chloride, and aluminium chloride and having a heat of solution greater than about 10 kcal/gram.mole, and a fragrance agent, the effervescent agent and exothermic agent provided in a ratio sufficient to promote release of the fragrance agent, when the composition is placed in water, the agents being in an essentially anhydrous, and placing the composition in water, wherein the ratio of effervescent agent to exothermic agent is from about 1:10 to about 10:1.

## Patentansprüche

1. Aroma freisetzende Tablette umfassend:
ein Brausemittel, wobei das Brausemittel eine Zusammensetzung ist, die beim Kontakt mit Wasser aufbraust:
ein exothermes Mittel, wobei das exotherme Mittel eine Zusammensetzung ist, die beim Kontakt mit Wasser eine exotherme Reaktion und eine Lösungswärme von mehr als ca. 10 kcal/Grammmol aufweist und von Magnesiumchlorid, Magnesiumsulfat, Eisenchlorid und Aluminiumchlorid ausgewählt wird; und
ein flüchtiges Duftmittel, wobei das Brausemittel und das exotherme Mittel in einem Verhältnis bereitgestellt werden, das ausreicht, um das Freisetzen des Duftmittels zu unterstützen, wenn die Zusammensetzung in Wasser eingegeben wird, wobei die Mittel in im Wesentlichen wasserfreier Form vorliege, wobei das Verhältnis von Brausemittel zu exothermen Mittel ca. 1 : 10 bis 10 : 1 beträgt.

2. Tablette nach Anspruch 1, wobei das Brausemittel eine Kombination von einem alkalischen Carbonatsalz und einer Säure ist.

3. Tablette nach Anspruch 2, wobei das alkalische Carbonatsalz aus der Gruppe ausgewählt wird bestehend aus Natriumcarbonat, Natriumbicarbonat, Natriumsesquicarbonat, Kaliumbicarbonat, Kaliumcarbonat, Kaliumsesquicarbonat, Magnesiumcarbonat, Ammoniumbicarbonat, Ammoniumcarbonat, Ammoniumsesquicarbonat, Calciumcarbonat und Kombinationen derselben.

4. Tablette nach Anspruch 2, wobei die Säure ausgewählt ist aus der Gruppe bestehend aus Zitronensäure, Äpfelsäure, Fumarsäure, Bernsteinsäure, Weinsäure Ameisensäure, Essigsäure, Propansäure, Buttersäure, Valeriansäure, Oxalsäure, Malonsäure, Weinsäure, Bernsteinsäure, Glutarsäure, Adipinsäure, Pimelinsäure, Fumarsäure, Maleinsäure, Phthalsäure, Isophthalsäure, Terephthalsäure, Glutaminsäure, Milchsäure, Hydoxyacrylsäure, Alphahydroxybuttersäure, Glycerinsäure, Tartronsäure, Hydroxybenzoesäure, Zitronensäure, Salicylsäure, Gallensäure, Mandelsäure, Tropasäure, Ascorbinsäure, Gluconsäure, Zimtsäure, Benzoesäure, Phenylessigsäure, Nicotinsäure, Kainsäure, Sorbinsäure, Pyrrolidoncarbonsäure, Trimellitsäure, Benzolsulfonsäure, Toluolsulfonsäure, Kaliumdihydrogenphosphat, Natriumsulfit, Natriumdihydrogenphosphat, Kaliumsulfit, Natriumpyrosulfit, saurem Natriumhexametaphosphat, saurem Kaliumhexametaphosphat, saurem Natriumpyrophosphat, saurem Kaliumpyrophosphat, Sulfamsäure, Phosphorsäure und Kombinationen derselben.

5. Tablette nach Anspruch 1, wobei das flüchtge Duftmittel aus der Gruppe ausgewählt ist bestehend aus Moschusöl, Zibet, Castoreum, Ambra, Pflanzenparfums wie Sandelholzöl, Neroliöl, Bergamottöl, Zitronenöl, Lavendelöl, Salbeiöl, Rosmarinöl, Pfefferminzöl, Eukalyptusöl, Menthol, Campher, Verbenaöl, Citronellöl, Cauoutöl, Salvienöl, Nelkenöl, Camillenöl, Sandelholzöl, Costusöl, Labdanumöl, Ginsterextrakt, Karottensamenextrakt, Jasminextrakt, Mimosenextrakt, Narzissenextrakt, Olibanumextrakt, Rosenextrakt und chemischen Substanzen wie Acetophenonen, Dimethylinadanderivaten, Napthalinderivaten, Allylcaprat, α-Amylcinnamaldehyd, Anethol, Anisaldehyd, Benzylacetat, Benzylalkohol, Benzylpropionat, Borneol, Cinnamylacetat, Cinnamylalcohol, Citralcitronellal, Cuminaldehyd, Cyclamenaldehyd, Decanol, Ethylbutyrat, Ethylcaprat, Ethylcinnamat, Ethylvanillin, Eugenol, Geraniol, Hexenol, α-Hexylcinnaminaldehyd, Hydroxycitronellal, Indol, Isoamylacetat, Isoamylisovalerat, Isoeugeol, Linalol, Linalylacetat, p-Methylacetophenon, Methylanthranilat, Methyldihydroasmonat, Methyleugenol, Methyl-β-naphtholketon, Methylphenylcarbinylacetat, Moschusketol, Moschusxylol, 2,5,6-Nanodinol, γ-Nanolacton, Phenylacetoaldehydodimethylacetat, β-Phenylethylalcohol, 3,3,5-Trimethylcyclohexanol, γ-Undecalacton, Undecenal, Vanillin und Mischungen derselben.

6. Tablette nach Anspruch 1, wobei das flüchtige Duftmittel aus der Gruppe ausgewählt ist bestehend aus Eukalyptusöl, Menthol, Campher und Kombinationen derselben.

7. Verfahren für das Freisetzen eines Aromas, umfassend: das Bereitstellen einer Aroma freisetzenden Tablette, die ein Brausemittel aufweist, das eine Zusammensetzung ist, die beim Kontakt mit Wasser aufbraust, ein exothermes Mittel, das eine Zusammensetzung ist, die beim Kontakt mit Wasser eine exotherme Reaktion aufweist, wobei das exotherme Mittel von Magnesiumchlorid, Magnesiumsulfat, Eisenchlorid und Aluminiumchlorid ausgewählt wird und eine Lösungswärme von mehr als ca. 10 kcal/Grammmol aufweist, und ein Duftmittel, wobei das Brausemittel und das exotherme Mittel in einem Verhältnis bereitgestellt werden, das ausreicht, um das Freisetzen des Duftmittels zu unterstützen, wenn die Zusammensetzung in Wasser eingegeben wird, wobei die Mittel in im Wesentlichen wasserfreier Form vorliegen, und das Eingeben der Zusammensetzung in Wasser, wobei das Verhältnis von Brausemittel zum exothermem Mittel ca. 1:10 bis 10:1 beträgt.

## Revendications

1. Comprimé à libération d'arôme, qui comprend :
- un agent effervescent, lequel agent effervescent est une composition qui entre en effervescence au contact de l'eau ;
- un agent exothermique, lequel agent exothermique est une composition qui donne une réaction exothermique au contact de l'eau, qui présente une chaleur de dissolution supérieure à environ 10 kcal/mol et qui est choisie parmi du chlorure de magnésium, du sulfate de magnésium, du chlorure ferrique et du chlorure d'aluminium ;
- et un agent odorant volatil ;
et dans lequel comprimé l'agent effervescent et l'agent exothermique se trouvent en des proportions suffisantes pour favoriser la libération de l'agent volatil quand on met la composition dans de l'eau, les agents sont sous forme pratiquement anhydre, et le rapport de l'agent effervescent à l'agent exothermique vaut d'environ 1/10 à environ 10/1.

2. Comprimé conforme à la revendication 1, dans lequel l'agent effervescent est une combinaison d'un sel alcalin de type carbonate et d'un acide.

3. Comprimé conforme à la revendication 2, dans lequel le sel alcalin de type carbonate est choisi dans l'ensemble constitué par les carbonate de sodium, bicarbonate de sodium, sesquicarbonate de sodium, carbonate de potassium, bicarbonate de potassium, sesquicarbonate de potassium, carbonate de magnésium, carbonate d'ammonium, bicarbonate d'ammonium, sesquicarbonate d'ammonium et carbonate de calcium, ainsi que leurs combinaisons.

4. Comprimé conforme à la revendication 2, dans lequel l'acide est choisi dans l'ensemble formé par les acides citrique, malique, succinique, tartrique, formique, acétique, propionique, butyrique, valérique, oxalique, malonique, glutarique, adipique, pimélique, fumarique, maléique, phtalique, isophtalique, téréphtalique, glutamique, lactique, hydroxy-acrylique, alpha-hydroxy-butyrique, glycérique, tartronique, hydroxy-benzoïque, salicylique, gallique, mandélique, tropique, ascorbique, gluconique, cinnamique, benzoïque, phényl-acétique, nicotinique, kaïnique, sorbique, pyrrolidone-carboxylique, trimellitique, benzène-sulfonique et toluène-sulfonique, les dihydrogénophosphate de potassium, sulfite de sodium, dihydrogénophosphate de sodium, sulfite de potassium, pyrosulfite de potassium, hexamétaphosphate acide de sodium, hexamétaphosphate acide de potassium, pyrophosphate acide de sodium et pyrophosphate acide de potassium, et les acides sulfamique et phosphorique, ainsi que leurs combinaisons.

5. Comprimé conforme à la revendication 1, dans lequel l'agent volatil est choisi dans l'ensemble formé par l'huile de musc, la civette, le castoréum, l'ambre gris, les parfums d'origine végétale comme les essences de bois de santal, de néroli, de bergamotte, de citron, de lavande, de sauge, de romarin, de menthe poivrée, d'eucalyptus, de verveine, de citronnelle, de cauout, de sauge sclarée, de girofle, de camomille, de costus et de labdanum, le menthol, le camphre, les extraits de fragon, de semences de carotte, de jasmin, de mimosa, de narcisse et de rose, et l'oliban, et des substances chimiques comme des dérivés de diméthyl-indane, des dérivés de naphtalène, et les acétophénone, caprate d'allyle, α-amyl-cinnamaldéhyde, anéthole, anisaldéhyde, acétate de benzyle, alcool benzylique, propionate de benzyle, bornéol, acétate de cinnamyle, alcool cinnamylique, citral, citronellal, aldéhyde de cumin, aldéhyde de cyclamen, décanol, butyrate d'éthyle, caprate d'éthyle, éthyl-vanilline, eugénol, géraniol, hexénol, aldéhyde α-hexyl-cinnamique, hydroxy-citronellal, indole, acétate d'isoamyle, isovalérate d'isoamyle, iso-eugénol, linalol, acétate de linalol, p-méthyl-acétophénone, anthranilate de méthyle, dihydrojasmonate de méthyle, méthyl-eugénol, méthyl-β-naphtyl-cétone, acétate de méthyl-phényl-carbinol, musc cétone, musc xylène, 2,5,6-nonadiénol, γ-nonalactone, acétal diméthylique de phénylacétaldéhyde, alcool β-phényl-éthylique, 3,3,5-triméthyl-cyclohexanol, γ-undécalactone, undécénal et vanilline, ainsi que leurs mélanges.

6. Comprimé conforme à la revendication 1, dans lequel l'agent volatil est choisi dans l'ensemble constitué par l'essence d'eucalyptus, le menthol et le camphre, ainsi que leurs combinaisons.

7. Procédé de libération d'arôme, qui comporte le fait de prendre un comprimé à libération d'arôme, comprenant un agent effervescent qui est une composition qui entre en effervescence au contact de l'eau, un agent exothermique qui est une composition qui donne une réaction exothermique au contact de l'eau, lequel agent exothermique est choisi parmi du chlorure de magnésium, du sulfate de magnésium, du chlorure ferrique et du chlorure d'aluminium et présente une chaleur de dissolution supérieure à environ 10 kcal/mol, et un agent odorant, l'agent effervescent et l'agent exothermique se trouvant en des proportions suffisantes pour favoriser la libération de l'agent odorant quand on met le comprimé dans de l'eau, et les agents étant sous forme pratiquement anhydre, et le fait de mettre le comprimé dans de l'eau, le rapport de l'agent effervescent à l'agent exothermique valant d'environ 1/10 à environ 10/1.
